(19) Europäisches Patentamt
European Patent Office
Office européen des brevets

(11) EP 2 756 250 B1

(12) EUROPÄISCHE PATENTSCHRIFT

(45) Veröffentlichungstag und Bekanntmachung des Hinweises auf die Patenterteilung:
16.09.2015 Patentblatt 2015/38

(21) Anmeldenummer: 12750528.7

(22) Anmeldetag: 07.08.2012

(51) Int Cl.:
*F27D 17/00* (2006.01)    *C07C 29/151* (2006.01)

(86) Internationale Anmeldenummer:
PCT/EP2012/003370

(87) Internationale Veröffentlichungsnummer:
WO 2013/037443 (21.03.2013 Gazette 2013/12)

(54) **VERFAHREN ZUR BEHANDLUNG VON KOKEREIABGAS**

METHOD FOR PROCESSING COKE OVEN GAS

PROCÉDÉ DE TRAITEMENT DES EFFLUENTS GAZEUX DE COKERIE

(84) Benannte Vertragsstaaten:
AL AT BE BG CH CY CZ DE DK EE ES FI FR GB GR HR HU IE IS IT LI LT LU LV MC MK MT NL NO PL PT RO RS SE SI SK SM TR

(30) Priorität: 15.09.2011  DE 102011113504
17.11.2011  EP 11009117

(43) Veröffentlichungstag der Anmeldung:
23.07.2014  Patentblatt 2014/30

(73) Patentinhaber: Linde Aktiengesellschaft
80331 München (DE)

(72) Erfinder:
• SCHÖDEL, Nicole
81477 München (DE)
• HAIDEGGER, Ernst
85521 Riemerling (DE)
• SCHMIGALLE, Holger (verstorben)
82538 Geretsried (DE)
• GÖKE, Volker
82538 Geretsried (DE)
• SCHMADERER, Harald
82515 Wolfratshausen (DE)

(74) Vertreter: Fischer, Werner
Linde AG
Legal Services
Intellectual Property
Dr.-Carl-von-Linde-Straße 6-14
82049 Pullach (DE)

(56) Entgegenhaltungen:
CN-A- 101 823 937    US-A1- 2011 112 314

**Beschreibung**

Die Erfindung betrifft ein Verfahren zur Verarbeitung von Abgas aus Kokerei, wobei das Abgas Wasserstoff enthält.

In einer Kokerei wird in einem Koksofen aus Kohle Koks erzeugt wird. Dabei werden in einem Koksofen die flüchtigen Bestandteile in der Kohle durch das Erhitzen auf eine Temperatur von 900 °C und 1400 °C pyrolysiert, freigesetzt und abgesaugt. Dabei entsteht der im Wesentlichen aus Kohlenstoff bestehende Koks und ein als Kokereigas bezeichnetes Abgas, welches die flüchtigen Bestandteile enthält. Die Pyrolyse im Koksofen findet in Abwesenheit von Sauerstoff statt. Es handelt sich prinzipiell um einen Batch-Prozess, wobei die Zusammensetzung des freigesetzten Kokereiabgases schwankt. Da jedoch stets eine Vielzahl von Kokssammlern betrieben wird, ist die mittlere Gaszusammensetzung nur kleinen fluktuationen unterworfen: Das entstandene Kokereigas enthält Wasserstoff (ca. 55%), Methan, Stickstoff, Kohlenmonoxid, Kohlendioxid, Schwefel und höhere Kohlenwasserstoffe.

WO 2009/023987, CN 101 913 558, CN 101 823 937 und CN 102 079 689 offenbaren Verfahren zum Herstellen von Methanol beziehungsweise Dimethylether aus Kokereiabgas und aus bei der Stahlherstellung anfallendem Abgas.

Die US 2011/0112314 beinhaltet ein Verfahren zur Herstellung von Olefinen aus sauerstoffhaltigen Einsatzprodukten.

Der vorliegenden Erfindung liegt die Aufgabe zugrunde, aus dem Abgas einer Kokerei ein oder mehrere Wertprodukte zu gewinnen.

Diese Aufgabe wird dadurch gelöst, dass das Abgas zumindest teilweise zusammen mit einem Kohlenmonoxid und/oder Kohlendioxid enthaltenden Gas in ein Verfahren zur Bildung von Methanol und/oder Dimethylether geführt wird, wodurch ein DMEhaltiges Produktgas gebildet wird, das DME-haltige Produktgas in ein Verfahren zur Umwandlung von Diemethylether zu Olefinen geführt wird, wodurch ein olefinhaltiges Produktgas gebildet wird, und wobei mittels Trennverfahren Olefine, insbesondere Ethylen und/oder Propylen, aus dem olefinhaltigen Produktgas abgetrennt wird/werden.

Weiterhin wird erfindungsgemäß am Eingang des Verfahrens zur Bildung von Dimethylether ein Verhältnis von Wasserstoff zu Kohlenmonoxid gewichtet mit der Kohlendioxidkonzentration $\dfrac{c[H2] - c[CO2]}{c[CO] + c[CO2]}$ von 0.9 bis 1.1, bevorzugt 1, eingestellt und Dimethylether gebildet. Vorteilhafterweise wird dabei auch Kohlendioxid aus Kohlenmonoxid gebildet.

Dabei der Wasserstoffgehalt derart reguliert, dass die Reaktion selektiv für Dimethylether abläuft, je nach dem weiteren konkreten Verfahren (Katalysator etc.) zur Bildung der Olefine, insbesondere Ethylen.

Der Grundgedanke der Erfindung besteht darin, aus dem wasserstoffhaltigen Abgas aus dem Koksofen einer Kokerei eine Art Synthesegas zu erzeugen und dieses in wertvolle Olefinprodukte umzuwandeln. Die Olefine können dabei mittels bekannter Trennverfahren aus dem olefinhaltigen Produktgas abgetrennt werden. Insbesondere kann das olefinhaltige Produktgas einfach als Einsatz in den Zerlegungsteil einer Olefinanlage geführt werden. Die vorliegende Erfindung lohnt sich dabei insbesondere an Standorten, wo günstige Kohlenmonoxid und/oder Kohlendioxid enthaltende Gas wirtschaftlich verfügbar sind.

Verfahren zur Umwandlung von beispielsweise Methanol in Olefine (z.B. Erzeugung von Ethylen durch katalytische Dehydrierung von Methanol über Aluminium und Zeolith Katalysatoren) sind im Stand der Technik bekannt und werden beispielsweise in "Ethylene", H. Zimmermann und R. Walzl in Ullmann's Encyclopedia of Industrial Chemistry, Wiley 2011 beschrieben. Gleiches gilt für die Abtrennung von Olefinen, insbesondere Ethylen und Propylen, aus derartigen olefinhaltigen Strömen (siehe gleiche Referenz und darin enthaltene Referenzen). Die vorliegende Erfindung ist jedoch nicht auf die dort beschriebenen Verfahren und die dort beschriebenen Trennverfahren limitiert.

Gemäß einer bevorzugten Ausgestaltung der Erfindung wird Abgas aus einem einem Hochofen und/oder einem Konverterofen eines Stahlwerks oder eines Hüttenwerks als Kohlenmonoxid und/oder Kohlendioxid enthaltendes Gas verwendet. Häufig befinden sich Kokereien in der Nähe von Stahl- oder Hüttenwerken, da in den Hochöfen derartiger Werke Koks in großen Mengen benötigt wird. In diesen Werken entstehen große Menge Kohlenmonoxid und/oder Kohlendioxid enthaltende Abgase, beispielsweise in den Hochöfen selbst oder in den Konverteröfen. In dieser Ausgestaltung der Erfindung kommen die erfindungsgemäßen Vorteile besonders zum Tragen, da hier die Abgase zweier Werkem in Wertprodukte umgewandelt werden.

Insbesonders Abgase aus einem Direktreduktionsverfahren für Eisenerz sind für das erfindungsgemäße Verfahren geeignet. Abgase aus dem Direktreduktionsverfahren für Eisenerz enthalten Kohelnmonoxid und Wasserstoff in einem Verhältnis zueinander, das zur Herstellung von Dimethylether ganz besonders geeignet ist.

In einer Ausgestaltung der Erfindung werden Abgas und/oder das Kohlenmonoxid und/oder Kohlendioxid enthaltende Gas aufgereinigt, bevor beide als Einsatz in das Verfahren zur Bildung von Methanol und/oder Dimethylether geführt werden. Dabei können beispielsweise aus dem Kohlenmonoxid und/oder Kohlendioxid enthaltende Gas alle Bestandteile bis auf Kohlenmonoxid und/oder Kohlendioxid entfernt werden. Das Abgas besteht nach der Aufreinigung zweckmäßigerweise nur noch aus Wasserstoff und optional Kohlenmonoxid und/oder Kohlendioxid.

Vorteilhafterweise das olefinhaltige Produktgas nach Abtrennung der Olefine als alkanhaltiges Restgas zur Unterfeuerung in den Koksofen und/oder Hochofen zurückgeführt wird. In den Abgasen der Öfen sind zum einen in geringem Anteil Kohlenwasserstoffe (vor allem Alkane) vorhanden, zum anderen werden in Nebenreaktionen bei der Olefinbildung auch Alkane gebildet. Nach Abtrennung der Olefine, insbesondere Ethylen und/oder Propylen, aus dem olefinhaltigen Produktgas besteht das alkanhaltige Restgas nunmehr hauptsächlich aus Alkanen und anderen brennbaren Bestandteilen. Daher ist es sehr gut zur Unterfeuerung der Öfen (Koksofen und/oder Hochofen) geeignet.

In einer Ausgestaltung wird aus dem alkanhaltigen Restgas Methan abgetrennt und als Einsatz in eine Gasturbine zur Erzeugung elektrischer Energie geführt wird. Diese Ausgestaltung der Erfindung kombiniert die Erfindung mit dem Stand der Technik beim dem das Abgas hauptsächlich zur Erzeugung elektrischer Energie verwendet wird.

Methan eignet sich von den Bestandteilen des Abgases am besten zur Verwendung in einer Gasturbine zur Erzeugung von elektrischer Energie und wird in dieser Ausgestaltung der Erfindung vom alkanhaltigen Restgas abgetrennt und als Einsatz in eine Gasturbine geführt oder in ein vorhandenes Erdgasnetz eingespeist.

In einer alternativen Ausgestaltung der Erfindung wird nach dem Verfahren zur Bildung von Dimethylether eine Fraktion aus DME-haltigen Produktgas abgetrennt, die Kohlenwasserstoffe mit höchstens einem Kohlenstoffatom enthält. Diese Fraktion besteht in dieser Ausgestaltung der Erfindung im Wesentlichen aus Methan:

Zweckmäßigerweise wird Wasserstoff mittels kryogener Trennverfahren aus dem olefinhaltigen Produktgas abgetrennt. Falls das olefinhaltige Produktgas noch Wasserstoff enthält, der in den vorhergehenden Verfahrensschritten nicht umgesetzt wurde, wird dieser mit der kryogenen Trennsequenz (beispielsweise bei Einspeisung des olefinhaltigen Produktgases in eine vorhandene Olefinanlage aber auch bei separater Trennsequenz) automatisch abgetrennt und kann als Produkt anderweitig in der Anlage verwendet oder ausgeführt werden.

In einer weiteren Ausgestaltung der Erfindung wird das alkanhaltige Restgas in ein Verfahren zur partiellen Oxidation von Alkanen in Alkene und Alkine unter Anwesenheit von Sauerstoff geführt, wodurch ein Oxidations-Produktgas entsteht, welcher in das Trennverfahren zur Abtrennung der Olefine zurückgeführt wird. Vorteilhafterweise wird der Wasserstoff und das Oxidations-Produktgas in ein Verfahren zur katalytischen Hydrierung von Alkinen geführt wird, wodurch ein Hydrier-Produktgas entsteht, und das Hydrier-Produktgas in das Trennverfahren zur Abtrennung der Olefine zurückgeführt wird.

Die beschriebenen Rückströme enthalten ebenfalls Olefine, insbesondere Ethylen und/oder Propylen, welche die Ethylen und/oder Propylen Ausbeute und somit die Wirtschaftlichkeit weiter erhöhen.

In einer anderen Ausgestaltung der Erfindung wird das alkanhaltige Restgas in ein thermisches Verfahren unter Sauerstoffabwesenheit geführt wird, wodurch ein Pyrolyse-Produktgas und Kohlenstoff entsteht, und wobei das Pyrolyse-Produktgas in ein Druckwechsel-Absorptionsverfahren geführt wird, wo es in Wasserstoff und ein acetylenhaltiges Restgas zerlegt wird. Das acetylenhaltige Restgas besteht nahezu überwiegend aus Acetylen, welches als Wertprodukt ausgeführt oder als Brennstoff in der Anlage verwendet werden kann. Neben der Verwendung eines Druckwechsel-Absorptionsverfahrens sind auch alternative, dem Fachmann vertraute Verfahren wie Membrantrennverfahren oder, insbesondere bei höhereen Acetylenghalten, auch chemische Wäschen beinhaltend mindestens eine Wasch- und Regenerationsstufe denkbar.

In einer weiteren Ausgestaltung der Erfindung wird das Kokereiabgas stromaufwärts des Verfahrens zur Bildung von Methanol und/oder Dimethylether in ein Verfahren zur Reformierung von Methan unter Bildung von Kohlenmonoxid geführt, wobei ein Reformer-Produktgas entsteht. In dieser Ausgestaltung der Erfindung wird der Kohlenmonoxidanteil im Eingang des Verfahrens zur Bildung von Methanol und/oder Dimethylether erhöht und somit die Produktbildung dieses Verfahrens begünstigt. Somit können im nachfolgenden Verfahrensschritt mehr Olefine, insbesondere Ethylen und/oder Propylen, gebildet werden. Zusätzlich wird der Methananteil im olefinhaltigen Produktgas geringer und somit die Abtrennung der Olefine, insbesondere Ethylen und/oder Propylen, vereinfacht. In einer alternativen Ausgestaltung kann der Reformer mit einem Wassergas-Shift Reaktor kombiniert werden.

Ebenso kann das alkanhaltige Restgas mit dem Abgas in das Verfahren zur Reformierung von Methan zur Erhöhung des Kohlenmonoxidanteils stromaufwärts des Verfahrens zur Bildung von Methanol und/oder Dimethylether rückgeführt werden.

Mit der vorliegenden Erfindung gelingt es insbesondere Kokereiabgase in Wertprodukte zu verwandeln. Das Abgas wird so nicht in Atmosphäre entlassen und die Umwelt entlastet.

**Patentansprüche**

1. Verfahren zur Verarbeitung von Abgas aus Kokerei, wobei das Abgas Wasserstoff enthält, **dadurch gekennzeichnet, dass** das Abgas zumindest teilweise zusammen mit einem Kohlenmonoxid und/oder Kohlendioxid enthaltenden Gas in ein Verfahren zur Bildung von Dimethylether geführt wird, wodurch ein DME-haltiges Produktgas gebildet wird, wobei am Eingang des Verfahrens zur Bildung Dimethylether ein Verhältnis von Wasserstoff zu Kohlenmonoxid gewichtet mit der Kohlendioxidkonzentration

$$\frac{c[H2]-c[CO2]}{c[CO]+c[CO2]}$$ von 0.9 bis 1.1 eingestellt wird, wobei

das DME-haltige Produktgas in ein Verfahren zur Umwandlung von Diemethylether zu Olefinen geführt wird, wodurch ein olefinhaltiges Produktgas gebildet wird, und wobei mittels Trennverfahren Olefine, insbesondere Ethylen und/oder Propylen, aus dem olefinhaltigen Produktgas abgetrennt wird/werden.

2. Verfahren nach Anspruch 1, **dadurch gekennzeichnet, dass** Abgas aus einem einem Hochofen und/oder einem Konverterofen eines Stahlwerks oder eines Hüttenwerks als Kohlenmonoxid und/oder Kohlendioxid enthaltendes Gas verwendet wird.

3. Verfahren nach Anspruch 1 oder 2, **dadurch gekennzeichnet, dass** Abgase aus einem Direktreduktionsverfahren für Eisenerz verwendet werden.

4. Verfahren nach einem der Ansprüche 1 bis 3, **dadurch gekennzeichnet, dass** am Eingang des Verfahrens zur Bildung von Dimethylether ein Verhältnis von Wasserstoff zu Kohlenmonoxid gewichtet mit der Kohlendioxidkonzentration

$$\frac{c[H2]-c[CO2]}{c[CO]+c[CO2]}$$ von 1, eingestellt wird.

5. Verfahren nach einem der Ansprüche 1 bis 4, **dadurch gekennzeichnet, dass** das olefinhaltige Produktgas nach Abtrennung der Olefine als alkanhaltiges Restgas als Einsatz in eine Gasturbine zur Erzeugung elektrischer Energie geführt wird.

6. Verfahren nach einem der Ansprüche 1 bis 5, **dadurch gekennzeichnet, dass** Wasserstoff mittels kryogener Trennverfahren aus dem olefinhaltigen Produktgas abgetrennt wird.

7. Verfahren nach einem der Ansprüche 1 bis 6, **dadurch gekennzeichnet, dass** das alkanhaltige Restgas in ein Verfahren zur partiellen Oxidation von Alkanen in Alkene und Alkine unter Anwesenheit von Sauerstoff geführt wird, wodurch ein Oxidations-Produktgas entsteht, und das Oxidations-Produktgas in das Trennverfahren zur Abtrennung der Olefine zurückgeführt wird.

8. Verfahren nach Anspruch 7, **dadurch gekennzeichnet, dass** der Wasserstoff und das Oxidations-Produktgas in ein Verfahren zur katalytischen Hydrierung von Alkinen geführt werden, wodurch ein Hydrier-Produktgas entsteht, und das Hydrier-Produktgas in das Trennverfahren zur Abtrennung der Olefine zurückgeführt wird.

9. Verfahren nach einem der Ansprüche 1 bis 6, **dadurch gekennzeichnet, dass** alkanhaltiges Restgas in ein thermisches Verfahren unter Sauerstoffabwesenheit geführt wird, wodurch ein Pyrolyse-Produktgas und Kohlenstoff entsteht, und wobei das Pyrolyse-Produktgas in ein Druckwechsel-Absorptionsverfahren geführt wird, wo es in Wasserstoff und ein acetylenhaltiges Restgas zerlegt wird.

10. Verfahren nach einem der Ansprüche 1 bis 9, **dadurch gekennzeichnet, dass** das Abgas stromaufwärts des Verfahrens zur Bildung von Dimethylether in ein Verfahren zur Reformierung von Methan unter Bildung von Kohlenmonoxid geführt wird, wobei ein Reformer-Produktgas entsteht.

**Claims**

1. Process for processing offgas from a coking plant, where the offgas contains hydrogen, **characterized in that** the offgas is at least partly fed, together with a gas containing carbon monoxide and/or carbon dioxide into a process for forming dimethyl ether, as a result of which a DME-containing product gas is formed, where a ratio of hydrogen to carbon monoxide weighted by the carbon dioxide concentration

$$\frac{c[H2]-c[CO2]}{c[CO]+c[CO2]}$$ of from 0.9 to 1.1 is set at the inlet of the process for forming dimethyl ether, the DME-containing product gas being fed to a process for converting dimethyl ether into olefins, as a result of which an olefin-containing product gas is formed, and olefins, in particular ethylene and/or propylene, being separated off from the olefin-containing product gas by means of a separation process.

2. Process according to Claim 1, **characterized in that** offgas from a steelworks comprising a blast furnace and/or a converter or from a smelting works is used as gas containing carbon monoxide and/or carbon dioxide.

3. Process according to Claim 1 or 2, **characterized in that** offgases from a direct reduction process for iron ore are used.

4. Process according to any of Claims 1 to 3, **characterized in that** a ratio of hydrogen to carbon monoxide weighted by the carbon dioxide concentration

$$\frac{c[H2] - c[CO2]}{c[CO] + c[CO2]}$$ of 1 is set at the inlet of the process for forming dimethyl ether.

5. Process according to any of Claims 1 to 4, **characterized in that** the olefin-containing product gas is, after separating off the olefins, fed as alkane-containing tail gas as feed to a gas turbine for generating electric energy.

6. Process according to any of Claims 1 to 5, **characterized in that** hydrogen is separated off from the olefin-containing product gas by means of a cryogenic separation process.

7. Process according to any of Claims 1 to 6, **characterized in that** the alkane-containing tail gas is fed into a process for the partial oxidation of alkanes to alkenes and alkynes in the presence of oxygen, as a result of which an oxidation product gas is formed, and the oxidation product gas is recirculated to the separation process for separating off the olefins.

8. Process according to Claim 7, **characterized in that** the hydrogen and the oxidation product gas are fed into a process for the catalytic hydrogenation of alkynes, as a result of which a hydrogenation product gas is formed, and the hydrogenation product gas is recirculated to the separation process for separating off the olefins.

9. Process according to any of Claims 1 to 6, **characterized in that** alkane-containing tail gas is fed to a thermal process in the absence of oxygen, as a result of which a pyrolysis product gas and carbon are formed, and wherein the pyrolysis product gas is fed into a pressure swing absorption process where it is separated into hydrogen and an acetylene-containing tail gas.

10. Process according to any of Claims 1 to 9, **characterized in that** the offgas is fed into a process for reforming methane to form carbon monoxide upstream of the process for forming dimethyl ether, forming a reformer product gas.

## Revendications

1. Procédé de traitement d'un effluent gazeux de cokerie, l'effluent gazeux contenant de l'hydrogène, **caractérisé en ce que** l'effluent gazeux est introduit au moins en partie conjointement avec un gaz contenant du monoxyde de carbone et/ou du dioxyde de carbone dans un procédé de formation d'éther diméthylique, par lequel un courant gazeux de produits contenant du DME est formé, un rapport entre l'hydrogène et le monoxyde de carbone pondéré par la concentration en dioxyde de carbone

$$\frac{c[H2] - c[CO2]}{c[CO] + c[CO2]}$$ de 0,9 à 1,1 étant ajusté à l'entrée du procédé de formation d'éther diméthylique, le courant gazeux de produits contenant du DME étant introduit dans un procédé de conversion d'éther diméthylique en oléfines, par lequel un courant gazeux de produits contenant des oléfines est formé, et des oléfines, notamment de l'éthylène et/ou du propylène, étant séparées du courant gazeux de produis contenant des oléfines par des procédés de séparation.

2. Procédé selon la revendication 1, **caractérisé en ce que** l'effluent gazeux d'un haut fourneau et/ou d'un four de conversion d'une aciérie ou d'une usine sidérurgique est utilisé en tant que gaz contenant du monoxyde de carbone et/ou du dioxyde de carbone.

3. Procédé selon la revendication 1 ou 2, **caractérisé en ce que** les effluents gazeux d'un procédé de réduction directe pour minerai de fer sont utilisés.

4. Procédé selon l'une quelconque des revendications 1 à 3, **caractérisé en ce qu'**un rapport entre l'hydrogène et le monoxyde de carbone pondéré par la concentration en dioxyde de carbone

$$\frac{c[H2] - c[CO2]}{c[CO] + c[CO2]}$$ de 1 est ajusté à l'entrée du procédé de formation d'éther diméthylique.

5. Procédé selon l'une quelconque des revendications 1 à 4, **caractérisé en ce que** le courant gazeux de produits contenant des oléfines est introduit en tant que charge sous forme d'un gaz résiduel contenant des alcanes après la séparation des oléfines dans une turbine à gaz pour la génération d'énergie électrique.

6. Procédé selon l'une quelconque des revendications 1 à 5, **caractérisé en ce que** l'hydrogène est séparé du courant gazeux de produits contenant des oléfines par des procédés de séparation cryogéniques.

7. Procédé selon l'une quelconque des revendications 1 à 6, **caractérisé en ce que** le gaz résiduel contenant des alcanes est introduit dans un procédé d'oxydation partielle d'alcanes en alcènes et alcynes en présence d'oxygène, par lequel un courant gazeux de produits d'oxydation est formé, et le courant gazeux de produits d'oxydation est recyclé dans le procédé de séparation pour la séparation des oléfines.

**8.** Procédé selon la revendication 7, **caractérisé en ce que** l'hydrogène et le courant gazeux de produits d'oxydation sont introduits dans un procédé d'hydrogénation catalytique d'alcynes, par lequel un courant gazeux de produits d'hydrogénation est formé, et le courant gazeux de produits d'hydrogénation est recyclé dans le procédé de séparation pour la séparation des oléfines.

**9.** Procédé selon l'une quelconque des revendications 1 à 6, **caractérisé en ce que** le gaz résiduel contenant des alcanes est introduit dans un procédé thermique en l'absence d'oxygène, par lequel un courant gazeux de produits de pyrolyse et du carbone sont formés, et le courant gazeux de produits de pyrolyse est introduit dans un procédé d'absorption modulée en pression, où il est décomposé en hydrogène et un gaz résiduel contenant de l'acétylène.

**10.** Procédé selon l'une quelconque des revendications 1 à 9, **caractérisé en ce que** l'effluent gazeux est introduit en amont du procédé de formation d'éther diméthylique dans un procédé de reformage de méthane avec formation de monoxyde de carbone, un courant gazeux de produits de reformeur se formant.

**IN DER BESCHREIBUNG AUFGEFÜHRTE DOKUMENTE**

**In der Beschreibung aufgeführte Patentdokumente**

- WO 2009023987 A
- CN 101913558
- CN 101823937
- CN 102079689
- US 20110112314 A

**In der Beschreibung aufgeführte Nicht-Patentliteratur**

- Ethylene. **H. ZIMMERMANN ; R. WALZL.** Ullmann's Encyclopedia of Industrial Chemistry. Wiley, 2011